# EUROPEAN PATENT SPECIFICATION

(11) **EP 3 298 101 B1**
(45) Date of publication and mention of the grant of the patent: **25.09.2019**
(21) Application number: 15778702.9
(22) Date of filing: 19.05.2015
(51) Int. Cl.: C02F 1/20, C02F 1/44, C02F 1/50, C02F 9/00, C09K 8/54, C09K 8/58, C12Q 1/04, E21B 43/20, G01N 33/487, C02F 101/10, C02F 103/10, C09K 8/52, E21B 41/02

(54) **WATER INJECTION SYSTEM COMPRISING BIOFILM SENSOR(S)**
WASSERINJEKTIONSSYSTEM MIT BIOFILMSENSOR(EN)
SYSTÈME D'INJECTION D'EAU COMPRENANT UN(DES) CAPTEUR(S) DE BIOFILM

(43) Date of publication of application: 28.03.2018
(73) Proprietor: Total SA, 92400 Courbevoie (FR)
(72) Inventor: BALDONI-ANDREY, Patrick, F-64320 Idron (FR); LESAGE, Nicolas, F-64140 Billere (FR); PEDENAUD, Pierre, F-64230 Lescar (FR); TOURNIS, Eric, F-64160 Morlaas (FR)
(74) Representative: Cabinet Plasseraud
(86) International application number: PCT/IB2015/001173
(87) International publication number: WO 2016/185245

(56) References cited:
- US-A1- 2004 120 853
- CYNTHIA DE ANDRADE ET AL: "REAL TIME MONITORING OF BIOFILMS IN SEAWATER INJECTION ENVIRONMENTS", CORROSION NACEXPO 2006, vol. 77084, 16 March 2006 (2006-03-16), XP055240566, California,USA

## Description

### Field of the invention

The present invention is in the field of water filtration for the purpose of enhanced oil recovery (EOR). More specifically, the invention concerns a water injection system for enhanced oil recovery that is provided with one or several biofilm sensors, as well as a method for removing a biofilm or controlling a biofilm formation within said system.

### Background

Water injection is an operation which consists in injecting water, commonly known as injection water, into an oil and gas well, in order to recover hydrocarbons and to avoid the wells collapsing, which can come about due to the drop in pressure as a result of the hydrocarbons being extracted.

The origin of the injection water generally depends on its availability and on the constraints around the site of the hydrocarbon extraction. In the case of offshore extraction, seawater is used. The injection water can also be aquifer water, river or lake water, as well as production water, domestic or industrial wastewater. In any case, treatment steps are generally necessary in order to obtain water which has a quality compatible with injection into the underground formation.

Thus, water injection treatment systems typically comprise a series of processes including filters of increasing selectivity along the water flow direction, from coarse filters to various membranes. Such a system is disclosed for example in US2004/0120853. Reverse osmosis offers the finest degree of separation, followed by nanofiltration, ultrafiltration, and microfiltration, which has the largest pore size. These filters or membranes are used to remove particles or to remove salts (desalination) from water.

For instance, when the injection water is seawater, the presence of sulfates in the water is typically a problem if the underground formation contains barium, calcium or strontium ions. Indeed, the sulfate ions form mineral deposits (scaling) with the barium, calcium or strontium ions and these are disadvantageous to good hydrocarbon extraction by reducing reservoir permeability. Furthermore, the presence of sulfates can be the cause of the generation by bacteria of hydrogen sulfide (H2S), a toxic and corrosive gas, which causes the souring of the natural gas, which in turn can lead to the corrosion of the pipes used for recovering hydrocarbons from the reservoir and a decrease of the commercial value of the gas. The elimination of the sulfates from the water before it is injected into the underground formation is therefore often necessary.

A conventional method enabling the removal of sulfates (desulfation) from the water consists in using a nanofiltration membrane that retains the multivalent ions and allows the monovalent ions to pass. Another conventional method enabling water desalination consists in using reverse osmosis membrane. Such methods are described, for example, in patent applications WO 2006/134367 and WO 2007/138327. The installation enabling desulfation of the water is generally called a sulfate removal unit (SRU).

Generally, the water treatment units such as SRUs are placed close to the hydrocarbon field. In the case of underwater fields, said units are conventionally installed on the surface, on the offshore platform for extracting hydrocarbons or on attached floating vessels that are called FPSO units (Floating Production Storage and Offloading units) for example. In order to save space on the FPSO unit or platforms, the water treatment unit may alternatively be placed and operated underwater (see for instance WO 2014/044976).

Like all material in contact with natural water, water treatment units tend to have their surface covered by a biofilm over time. This phenomenon is called "biofouling". It corresponds to the undesired accumulation and growth of microorganisms, algae and animals on the exposed surfaces of the material. Biofouling is initiated by "micro-fouling", i.e. the growth of a group of microorganisms such as bacteria or microalgae that are embedded in a matrix containing organic matter and dissolved macromolecules, such as polysaccharides, proteins and protein fragments, all this together constituting a biofilm. The biofilm then becomes a substrate for "macro-biofouling", i.e. the colonization of larger organisms, such tunicates, balanides, mollusks and algae.

After a certain amount of time, biofouling can form substantial incrustations that can cause the total or partial clogging of some elements of the water treatment unit, especially filters or membranes, or render some elements considerably heavier, or can also accelerate the corrosion of metals.

The fouling of membranes can also be caused by organic deposits resulting from the accumulation of residual fragments of organic cells or algae generated during chlorination of the water flow. These deposits are not caused by bacterial growth but result from the decomposition of said organic cells or algae.

The lifetime and efficiency of filters, membranes or other water treatment processes in water injection systems can be significantly reduced if the biofouling is not prevented or controlled.

In order to prevent or control the biofouling of a filter or a membrane, the water flow is generally treated before it reaches said filter or membrane, either by injecting a biocide in amounts sufficient to kill all bacteria present in the water flow or by passing the water flow through pre-filters able to retain bacteria or most often by combining the two means. In SRUs, fine filtration units such as media filtration combined with cartridge filtration or porous membranes such as microfiltration membranes or ultrafiltration membranes are typically placed upstream the desulfation membrane in order to remove suspended solids and microorganisms present in the water flow. The biocide treatment is generally performed by periodically injecting a pre-determined dose of biocide in the water flow, most often chlorine compounds such a sodium hypochlorite or DBNPA (2,2-dibromo-3-nitrilopropionamide) which is a non-oxidizing biocide.

Since the biofouling phenomenon is strongly influenced by the geographic characteristics of the site that hosts the structure, the temperature and hydrodynamics of the water and on the roughness of the surface of the structure, the frequency of injection and the concentration of the biocide are generally determined based on acquired experience.

Bacterial cells range from about 1 to 10 µm in length and from 0.2 to 1 µm in width while protozoan cysts are typically 2 to 50 µm in diameter. Therefore, in order to be able to retain microorganisms from the water flow, the fine filtration units in water injection systems are generally selected among those having a pore size of 0.1 µm or lower. Those fine filtration units act as a physical barrier against microorganisms that may form a biofilm.

By implementing the above described water pretreatments to the water flow, the experience shows that it is possible to control or prevent the development of biofilm on membrane filtration units in a water injection system in a satisfactory manner during many years without any intervention whatsoever, during at least 8 years.

Surprisingly, the inventors of the present application has evidenced that a biofilm can still develop downstream the fine filtration units despite their action as physical barriers against microorganisms. What is more startling is that under certain conditions, the biofilm can grow faster downstream the fine filtration units than upstream. In particular, this may occur if the water velocity at the entrance of the fine filtration unit is higher than at the outlet. To the Applicant's knowledge, this problem has never been recognized.

This problem is a major issue because SRU membranes, such as nanofiltration membranes or reverse osmosis membranes, which are located downstream the fine filtration units are difficult to clean-up due to their spiral wound configuration which does not allow backwash. Moreover, in-situ visual observation is not possible. In order to assess the development of a biofilm, the membrane has to be open (destructive analysis). Moreover, it cannot be performed while the system is being operated. As of today, the biofilms are detected when they induce a loss of efficiency, such as a loss of permeability, an increase of the pressure drop, an increase of head loss, an increase of temperature in a heat exchanger or a build-up of solids at some locations. At that time, the biofilm is well developed and thus is more difficult to remove.

Thus, a monitoring of the biofilm development at the exit of the fine filtration unit appears to be critical for protecting downstream SRU membranes from biofouling. If a biofilm is detected in the filtrate exiting the fine filtration unit, then a dose of biocide can be injected at an early stage of the biofilm development which facilitates the removal of the biofilm. Furthermore, the dose of biocide can be precisely determined depending on the growth level of the biofilm, which makes it possible to avoid the excess of biocide because some biocides may be detrimental to the membrane. The biofilm sensor of the invention also ensures that a biofilm has been efficiently removed either by injecting a biocide or by using a cleaning-in-place system. Therefore, a monitoring of the biofilm growth in the filtrate exiting fine filtration units, especially a real-time monitoring, allows a very efficient protection of downstream membranes, such as SRU membranes, as well as optimization of the biocide treatment (choice of the biocide, in which concentration, how often the injection is made, mode of injection: in batch or in continuous). It also helps in optimizing the cleaning and operation of the filters and membranes (mode of injection of chemicals, how often, time of replacement of the filtration unit), or even avoid the use of cleaning compounds, thus bringing an economic and environmental gain.

### Summary of the invention

A first object of the invention is a water injection system for enhanced oil recovery that is provided with one or several biofilm sensors in continuous contact with the water flow.

The water injection system of the invention comprises in the direction of water flow:
- a water intake for bringing a flow of water taken from the environment into said water injection system;
- a biocide tank for injecting a dose of biocide into said flow of water,
- a fine filtration unit able to retain particles of a size of 0.1 µm or higher,
- a biofilm sensor in continuous contact with the water flow,
- a water injection line.

A second object of the invention is a method for removing a biofilm or controlling the formation of a biofilm on the surface of the pipes or the filtering elements of the water injection system for enhanced oil recovery as defined above.

The method of the invention comprises the following steps:
- providing the water injection system with a biofilm sensor in continuous contact with the water flow, said biofilm sensor being located downstream the fine filtration unit,
- measuring the biofilm growth response R of the biofilm sensor,
- comparing said biofilm growth response R with a reference value Ro,
- if R is higher than Ro, injecting a dose of biocide into the flow of water from the biocide tank and/or changing the operating conditions of the fine filtration unit.

In one embodiment, the water injection system further comprises a desalination unit located downstream the biofilm sensor and upstream the water injection line.

In one embodiment, the desalination unit comprises a desulfation membrane.

In one embodiment, the desulfation membrane comprises a nanofiltration membrane, a reverse osmosis membrane or a combination thereof.

In one embodiment, the fine filtration unit comprises a media filtration unit, a cartridge filtration unit, a microfiltration membrane or an ultrafiltration membrane, or a combination thereof.

In one embodiment, the water injection system further comprises coarse filter(s) located upstream the fine filtration unit.

In one embodiment, the water injection system further comprises a deaeration unit located upstream or downstream the fine filtration unit or upstream or downstream the desalination unit but always upstream the water injection line.

In one embodiment, the water injection system further comprises a biofilm sensor located downstream the desalination unit and upstream the water injection line.

In one embodiment, the water injection system is connected to an injection wellhead and a biofilm sensor is located at the injection wellhead.

In one embodiment, the biofilm sensor is an electrochemical probe, such as an electrochemical probe whose operation is based on the cathodic depolarization induced by the biofilm growth or an electrochemical impedance probe, or a probe operating on differential turbidimetry, light scattering, heat transfer, pressure-drop, real-time measurement of metabolic products, image analysis, radiation signals or electric signal or vibration signals.

In one embodiment, the water injection system is an entire subsea system or is placed on a floating vessel or a platform or onshore.

### Brief description of the figures

Figure 1 is a graph representing the evolution of a biofilm sensor signal (mV) as a function of the sodium hypochlorite concentration (from 0.1 ppm to 2 ppm). Dotted lines show the detection level (bottom line) and saturation level (upper line).
Figure 2 shows the calibration curve of sodium hypochlorite derived from the peak heights of Figure 1.
Figure 3 is a graph representing the evolution of a biofilm sensor signal as a function of time. Continuous line represents system subjected to sodium hypochlorite injection. Dotted line represents system subjected to DBNPA injection. Dots represent the temperature of the water. Arrows show the time at which the biocide is injected. Gray area corresponds to an artifact due to a leak into the sodium hypochlorite system and must not be interpreted.
Figure 4 is a schematic representation of the pilot used at example 2.3).
Figure 5 is a graph representing the evolution of a biofilm sensor signal as a function of time of probe 2 (dotted line) and probe 1 (continuous line). Dots represent the temperature of the water. Arrows show the time at which the dose of sodium hypochlorite is injected (2 ppm).
Figure 6 is a graph representing the evolution of a biofilm sensor signal as a function of time of probe 2 (dotted line) and probe 1 (continuous line) in steady-state operation. Dots represent the temperature of the water. Arrows show the time at which the dose of sodium hypochlorite is injected (first injection: 50 mg/L for 2 hours; second injection: 50 mg/L for 1 hour).
Figure 7 is a schematic representation of a water injection system according to the invention.

### Detailed description of the invention

A first object of the invention is a water injection system for enhanced oil recovery that is provided with one or several biofilm sensors in continuous contact with the water flow.

The water injection system of the invention is used for treating water withdrawn from the environment in such a way that said water is suitable for being injected into a hydrocarbon field. The term "environment" means not only the natural environment (water may for example be withdrawn from water streams or surface water expanses notably rivers, lakes and the sea, or further may be withdrawn from an underground water-bearing formation), but also non-natural sources of water, such as produced water from an oil and gas reservoir or industrial wastewater.

In a preferred embodiment, the water is withdrawn from sea.

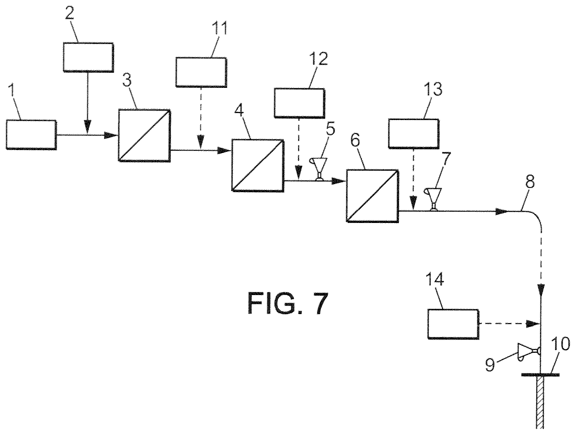

Figure 7 is a schematic representation of a water injection system according to the invention.

With reference to Figure 7, the water injection system of the invention comprises in the direction of water flow:
- a water intake 1 for bringing a flow of water taken from the environment into said water injection system;
- a biocide tank 2 for injecting a dose of biocide into said flow of water,
- a fine filtration unit 4 able to retain particles of a size of 0.1 µm or higher,
- a biofilm sensor 5 in continuous contact with the water flow,
- a water injection line or network 8.

As described below, the water injection system of the invention may also comprise a coarse filter 3 and a desalination unit 6.

The flow of water enters water treatment system via a water intake 1. A water intake is a conventional system which includes a pumping system enabling a flow of water to enter the system. The water intake 1 can be mobile, for example by means of a telescopic system which enables the site of the water intake to be varied without modifying the location of the water injection system unit itself. In particular, a telescopic system or a rewinder can allow the level of the water intake to be moved.

The water intake can be provided with a strainer screen (not represented on Figure 7) which holds back large diameter solid elements, thus avoiding the filtration system quickly becoming blocked by large elements. Between the strainer screen and the fine filtration units, the water injection system can include other filters 3, such as a sieve or a pre-filter enabling coarse filtration. The term "pre-filter" generally designates a filter that is able to stop relatively large-sized solid particles, above 100 µm.

The biocide tank 2 can be any reservoir suitable for containing an amount of biocide and for injecting a dose of biocide into the water flow from this reservoir. The reservoir may be rigid or flexible. There is no limitation regarding the form of the biocide contained in the tank 2: it is preferably in liquid form, but it can also be in solid form, such as a powder or a tablet, as long as it is injectable. The biocide tank 2 comprises an injector allowing injection of the biocide into the water flow either in batch or continuously. The biocide tank 2 is preferably provided with a doser, such as a Venturi dosing device or dosing pump. The biocide tank 2 is preferably positioned nearby the water inlet upstream the coarse filters.

As represented in Figure 7, the water injection system of the invention may further comprise one or more additional biocide tanks for injecting a biocide, identical to or different from the biocide of tank 2, into the water flow in other parts of the system. For instance, an additional biocide tank 11 may be positioned upstream the fine filtration unit 4. Furthermore, when a desalination unit 6 is present, an additional biocide tank 12 may be positioned upstream the desalination unit 6 and downstream the fine filtration unit 4. Furthermore, an additional biocide tank 13 may be positioned upstream the injection line 8. Furthermore, an additional biocide tank 14 may be positioned at the wellhead 10.

According to the invention, a biocide is a chemical substance which is able to remove the biofilm, kill the biofilm cells or limit the growth or attachment of microorganisms. The biocide can be chosen from oxidizing compounds, such as hydrogen peroxide or chlorinated compounds, in particular sodium hypochlorite, or from non-oxidizing compounds, such as DBNPA (dibromo nitrolo proprionamide), isothiazoline, sulfathiazole or glutaraldehyde.

Chlorine compounds and any oxidizing are a hazard for the chemical structure of SRU membranes. Therefore, chlorinated or oxidizing biocides are preferably injected at the furthest possible point upstream of the system. When the system comprises a desalination unit 6, a dechlorination treatment may be required upstream the desalination unit 6. The dechlorination treatment may be performed by using a deaeration unit or chemicals.

The fine filtration unit 4 is chosen among filtration systems able to retain particles of a size of 0.1 µm or higher, including microorganisms such as bacteria or protozoan cysts that may form a biofilm. Such filtration systems may be chosen among filtration membranes possibly in combination with media filters.

Filtration membranes are porous devices which enable different components of a liquid flow to be separated. Membranes come in four basic configurations: tubular, spiral, hollow fiber, and flat sheet. The nature of the separation is determined in part by the dimension of the pores in the membranes.

According to the IUPAC classification:
- a microfiltration membrane has macropores the diameter of which is in excess of 50 nm,
- an ultrafiltration membrane has mesopores the diameter of which is between 2 nm and 50 nm inclusive,
- a nanofiltration membrane has micropores the diameter of which is less than 2 nm.

Filtration membranes also include reverse osmosis (RO) membranes. These membranes feature the smallest pores and involve the reversal of osmotic pressure in order to drive water away from dissolved molecules. Strictly speaking, RO is not a size exclusion process based on pore size; it depends on ionic diffusion to affect separation. One of its common applications is seawater desalination, in which pure water is produced from a highly saline feed stream, similar to evaporation with far better economy.

In the water injection system of the invention, media filters and membranes can be arranged in series and/or in parallel. The expression "fine filtration unit" denotes a unit comprising one or several filters arranged in series and/or in parallel. The expression "membrane" denotes one or several membranes arranged in series and/or in parallel.

In one embodiment, the fine filtration unit 4 is a media filter combined with cartridge filters.

In one embodiment, the fine filtration unit 4 is an ultrafiltration membrane or a microfiltration membrane.

As previously discussed, the presence of sulfates in water may cause sulfate scale formation in the oil reservoir during water injection, as well as the souring of the gas, which in turn can lead to the corrosion of the pipes used for recovering hydrocarbons from the reservoir. The elimination of the sulfates or other salts from the water before it is injected into the underground formation is therefore often necessary. To this end, a desalination unit may be used.

In one embodiment, the water injection system of the invention further comprises a desalination unit 6 located downstream the biofilm sensor 5 and upstream the water injection pipe 8.

In one particular embodiment, the desalination unit 6 comprises a desulfation membrane.

The expression "desulfation membrane" refers to a membrane which enables sulfate ions to be separated from water. The desulfation membrane may comprise a nanofiltration membrane, a reverse osmosis membrane or a combination thereof. These membranes are able to retain not only sulfate ions but also other ions such as calcium ions, magnesium ions, iron ions or carbonate ions.

In a preferred embodiment, the desulfation membrane is a spiral-wound nanofiltration membrane.

Among the nanofiltration membranes for the desulfation of seawater, the membranes SR90 of DOW Filmtec and the NANO-SW of Hydranautics can be cited, among others.

According to the invention, the water injection system comprises a biofilm sensor 5 located downstream the fine filtration unit 4, for measuring the biofilm which may develop in the filtrate exiting the fine filtration unit 4. Preferably, the biofilm sensor 5 is positioned nearby the exit of the fine filtration unit 4 or directly upstream the desalination unit 6 when present. As explained previously, the inventors have evidenced that despite the presence of the fine filtration units 4 which are supposed to physically retain the microorganisms, a biofilm may still develop downstream the fine filtration units. Thus, the monitoring of the biofilm development at the exit of the fine filtration unit 4 appears to be critical for protecting downstream equipments and filtration units 4, such as SRU membranes, from biofouling.

According to the invention, a biofilm sensor is a sensor able to detect or quantify a biofilm growth on a surface. The biofilm sensor 5 is an intrusive probe, i.e. it is attached to the water injection system in such a way that the sensitive part of the biofilm sensor is immersed into the flow of water (directly inline or on a derivation loop). Preferably, the probe allows real-time monitoring of the biofilm growth. The measurement can be done continuously or at chosen time intervals.

In one embodiment, the biofilm sensor 5 is adapted for transmitting the measured data to a remote receptor.

In one embodiment, the biofilm sensor 5 is provided with a display for enabling graphical visualization of the measured data.

In one embodiment, an alarm is activated when the biofilm covering exceeds a given threshold.

In one embodiment, the biofilm sensor 5 is adapted for transmitting information to the injector of the biocide tank 2, 11, 12, 13 or 14.

In one embodiment, the biofilm sensor 5 is adapted for transmitting information to the Distributed Control System of the water injection system that enables the control of the operating conditions of each unit of the system.

The biofilm sensor 5 can operate on various sensing techniques. For instance, it can be an electrochemical probe or a probe operating on differential turbidimetry, light scattering, heat transfer, pressure-drop, real-time measurement of metabolic products, image analysis, radiation signals or electric signal or vibration signals. The radiation signal may include spectroscopy, fluorometry or photoacoustic spectroscopy. See for instance Janknecht et al. (2003) for a review of online biofilm monitoring systems. All online biofilm monitoring techniques are based on some kind of signal obtained from the biofilm under investigation. Input signals are transmitted to the investigated surface, modified by the biofilm (if present) and its environment, detected by specific sensors and usually processed in more or less sophisticated ways. Signal features that may be modified by the biofilm include:
- intensity of light (Differential turbidity);
- intensity of sound (Ultrasonic Frequency-Domain Reflectometry);
- color/wavelengths (Bioluminescence, Flurometry, Spectroscopy);
- mechanical resonance frequencies (Quartz Crystal Microbalance);
- electrical capacitance (Dielectric sensor);
- electrical conductivity (Electrochemical Electrodes);
- light refraction indices (Surface Plasmon Resonance);
- friction (Pressure Drop);
- thermal resistance (Heat Transfer Coefficient);
- optical input signals that are being modified into acoustic output signals (Photoacoustic Spectoscopy).

In one particular embodiment, the biofilm sensor 5 is an electrochemical probe which measures the biofilm electrochemical signal (BES, expressed as current density or potential). It was proven that BES is proportional to the surface area covered by bacteria. In one particular embodiment, the biofilm sensor 5 is chosen among electrochemical probes whose operation is based on the cathodic depolarization induced by the biofilm growth or electrochemical impedance probes.

For instance, the biofilm sensor 5 can be an ALVIM Biofilm Monitoring sensor provided by ALVIM Srl. This biofilm sensor allows real-time and continuous monitoring of bacterial covering even at early stages of colonization. It is able to detect the electrochemical activity of a biofilm (Faimali et al., 2010 and Pavanello et al., 2011).

When the water injection system comprises a desalination unit 6, in particular a SRU membrane, a further biofilm sensor 7 may be positioned downstream the desalination unit 6 and upstream the water injection lines 8.

The water injection system may also comprise a further biofilm sensor 9 located at the injection wellhead 10 of the reservoir.

In a particular embodiment, the water injection system comprises:
- a water intake 1 for bringing a flow of water taken from the environment into said water injection system;
- a biocide tank 2 for injecting a dose of biocide into said flow of water,
- a fine filtration unit 4 able to retain particles of a size of 0.1 µm or higher, in particular an ultrafiltration unit;
- a first biofilm sensor 5 in continuous contact with the water flow,
- a desalination unit 6, in particular a sulfate removal unit comprising a reverse osmosis membrane or a nanofiltration membrane,
- a second biofilm sensor 7 in continuous contact with the water flow,
- a water injection line 8,
- a third biofilm sensor 9 in continuous contact with the water flow located at the injection wellhead 10.

The water injection system of the invention may also comprise a deaeration unit (not represented in Figure 7) for removing oxygen molecules from the water flow, thereby reducing corrosion and the development of bacteria. This unit may also allow the elimination of residual chlorine. The deaeration unit may be located between the fine filtration unit 4 and the desalination unit 6 or downstream the desalination unit 6 and upstream the injection lines 8 in order to limit the souring of the gas in the oil reservoir.

The water injection system of the invention may also a comprise a cleaning system for the filters and membranes (not represented in Figure 7), such as back-wash systems, air scour systems, chemically enhanced back wash systems (with acids or bases) or cleaning-in-place systems.

In order to make the water circulate across the water injection system, the water injection treatment system of the invention includes at least one pump (not represented in Figure 7) which is suitable for filtering water. Said pump can be situated on the pipe which connects the water intake and the fine filtration unit. The pump can be controlled by an electronic system. Said electronic system can be controlled by a pre-recorded program which does not require the intervention of an operator. As an alternative to this, it can be controlled by an operator and the information exchanged between the operator and the electronic system can be transmitted via cable or online (for example, over the air, notably wirelessly or acoustically in the water if the water injection system is underwater).

The water injection line 8 downstream of the fine filtration unit 4 is used to feed the injection well with the filtrate recovered from the system. Said filtrate constitutes treated water which is suitable to be used as injection water in a hydrocarbon field. To this end, the filtrate injection line 8 is connected to an injection wellhead 10. The water injection system of the invention may comprise one or several water injection lines 8 for feeding several injection wellheads. The water treatment system of the invention can also include discharge pipe(s) for the retentate(s) (not represented in Figure 7).

The water injection system of the invention may be applied on land (onshore) or at sea (offshore). The offshore application may be on a floating vessel (FPSO) or a platform, or further on the sea bed, underwater, while using suitable equipment (marinization of the equipment). In particular, the water injection system may be an entire subsea system.

In order to be operated, the water injection system of the invention needs a source of energy. This is why the water injection system of the invention of course includes a power supply (not represented in Figure 7).

A second object of the invention is a method for removing a biofilm or controlling the formation of a biofilm on the surface of the pipes or the filtering elements of the water injection system for enhanced oil recovery as defined previously.

The method of the invention comprises the following steps:
- providing the water injection system with a biofilm sensor 5 in continuous contact with the water flow, said biofilm sensor being located downstream the filtration unit 4,
- measuring the biofilm growth response R of the biofilm sensor 5,
- comparing said biofilm growth response R with a reference value Ro,
- if R is higher than Ro, injecting a dose of biocide into the flow of water from the biocide tank 2 or 11 and/or changing the operating conditions of the fine filtration unit 4.

Changing the operating conditions of fine filtration unit 4 means that an action is performed so that the filtration conditions are changed. For instance, the number of filters in operation may be changed or the cleaning of a filter may be triggered or the flow rate going through the filter may be changed.

The biofilm sensor 5 allows detection of the formation of a biofilm in the filtrate of the fine filtration unit 4 at early stage of development and helps to ensure that the biofilm is removed.

The reference value Ro is the value at which the biofilm level is considered undesirable and needs to be removed. For instance, with ALVIM probes, Ro may be from 0.1% to 1 % of the working electrode but other ranges could be chosen by the operator based on field experience of the probe into the considered system.

In one embodiment, when R is higher than Ro, an alarm is activated thereby triggering injection a dose of biocide into the flow of water from the biocide tank 2 or 11. The injection may be performed manually by a practitioner or automatically by transmitting the information from the biofilm sensor to the injector of the biocide tank 2 or 11.

The dose of biocide injected into the water flow can be a predetermined dose or can be adjusted depending on the response R of the biofilm sensor. The response R may be displayed on a screen or be transmitted to a receptor able to automatically trigger an injection of biocide into the water flow.

In one embodiment, the injection of the biocide is done during a certain period of time until the biofilm growth response R reaches a reference value Roₘᵢₙ, wherein Roₘᵢₙ is the value at which the biofilm level is considered acceptable. The injection may be done continuously or in batches.

In one embodiment, when R is higher than Ro, the operating conditions of the fine filtration unit 4 are changed. For instance, when the fine filtration unit 4 comprises several filters and/or several membranes, the cleaning of a filter or a membrane may be triggered, thus the number of filters/membranes in operation is reduced and the flow rate going through each filter/membrane in operation is increased. In case of membranes, the flow rate treated by each train of membranes can be reduced in order to delay the biofouling of the membranes, or the pressure can be increased in order to maintain the water flow rate through the membranes. The change of operating conditions may be performed manually by the operator or automatically by transmitting the information from the biofilm sensor to the Distributed Control System of the water injection system.

As indicated previously, the water injection system of the invention may comprise several biofilm sensors located at various places of the system:
- a first biofilm sensor 5 between the fine filtration unit 4 and the desalination unit 6, such as a sulfate removal unit,
- a second biofilm sensor 7 between the desalination unit 6 and the water injection line 8,
- a third biofilm sensor 9 located at the injection wellhead 10.

In one embodiment, the method of the invention further comprises the following steps:
- providing the water injection system with a further biofilm sensor 7 located downstream the desalination unit 6 and upstream the water injection line 8,
- measuring the biofilm growth response Ra of the further biofilm sensor 7,
- comparing said biofilm growth response Ra with a reference value Rao,
- if Ra is higher than Rao, injecting a dose of biocide into the flow of water from the biocide tank 2, 11 or 12 and/or changing the operating conditions of the fine filtration unit 4 and/or the desalination unit 6.

The injection of a dose of biocide may be performed as described previously.

The change of operating conditions of the fine filtration unit 4 and/or the desalination unit 6 may be performed as described previously.

In one embodiment, the water injection system of the invention is connected to an injection wellhead and said method further comprises:
- providing the water injection system with a further biofilm sensor 9 located at the injection wellhead 10,
- measuring the biofilm growth response Rb of the further biofilm sensor 9,
- comparing said biofilm growth response Rb with a reference value Rbo,
- if Rb is higher than Rbo, injecting a dose of biocide into the flow of water from the biocide tank 2, 11, 12, 13 or 14 and/or changing the operating conditions of the fine filtration unit 4 and/or the desalination unit 6.

The injection of a dose of biocide may be performed as described previously.

The change of operating conditions of the fine filtration unit 4 and/or the desalination unit 6 may be performed as described previously.

The following examples provide another illustration of the invention but without restraining to the scope of the invention.

### Examples

### 1) Equipment and pilot facility

Tests of biofilm detection were carried out in a pilot plant continuously fed with Mediterranean seawater (2m deep, 50 m from the coast). The experimental conditions are favorable to the biofilm growth: laminar flowing and coastal seawater rich in microorganisms and suspended solids (Silt Density Index SDI₅ₘᵢₙ = 10-18).

Tests were carried out in a tank divided in 6 parallel channels with equal dimensions (0.76 m-long, 0.1 m-deep and 0.07 m-wide per channel). Each channel was continuously fed with from 10 L/h to 40 L/h of seawater pre-filtered with a multimedia filter (40µm). The speed of water flowing in the channels ranges from 0.0003 to 0.0013 m/s.

This set up allows the testing of several biocides and their comparison on the same sea water quality. Various biocide injection strategies can also be compared such as continuous or batch injection upstream the channels.

Several channels are equipped with an electrochemical biofilm sensor whose working electrode is immersed within the flowing water phase. The electrochemical biofilm sensor is provided by ALVIM Srl. This biofilm sensor is able to detect the electrochemical activity of a biofilm (Faimali et al., 2010 and Pavanello et al., 2011). This activity is proportional to the surface covered with biofilm. Thus, when a biofilm appears on the flat surface of the working electrode, the electrical signal transmitted by the device instantly increases. When the working electrode is immersed in seawater, its potential is stable (around 600 mV). Whenever the working electrode detects the biofilm, its potential increases and reaches a potential threshold which relate to a given maximum value of the probe (signal saturation).

### 2) Results

### 2.1) Impact of sodium hypochlorite injections on the probe signal

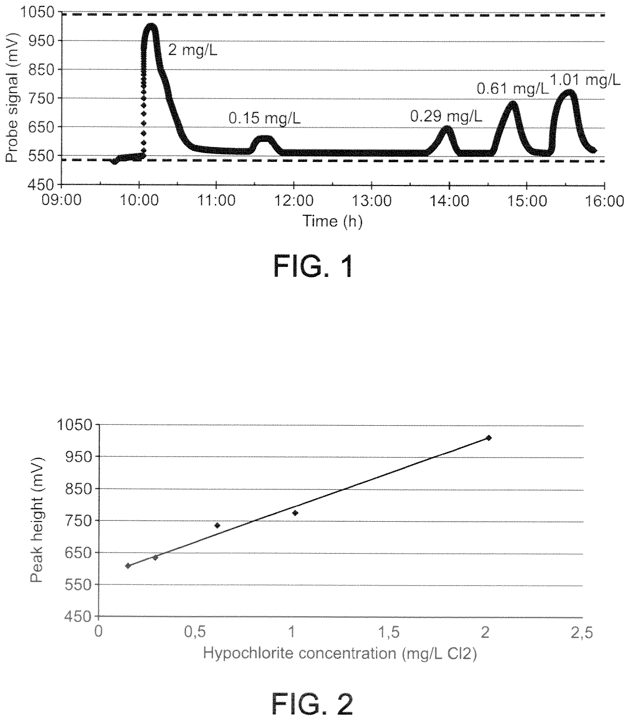

A series of tests was performed by injecting various doses of sodium hypochlorite (ClO⁻Na+) upstream the channel. Figure 1 shows the evolution of the signal (mV) of an ALVIM probe as a function of the sodium hypochlorite concentration (from 0.1 ppm to 2 ppm). Dotted lines show the detection level (bottom line) and saturation level (upper line). A linear response of the probe up to 2 mg/L can be expected in those experimental conditions where the highest peak is close to the saturation level. Figure 2 shows the calibration curve of sodium hypochlorite derived from the peak heights of Figure 1 (peak heights as a function of measured sodium hypochlorite content; y = 212.52 x + 583.24; R² = 0.9882).

These results show that the ALVIM probe is able to detect sodium hypochlorite which is an oxidizing agent.

The same probes have been exposed to injections of various doses of non-oxidizing biocides such as DBNPA and Isothiazoline. Up to 100 mg/L, no signal was observed. It can be concluded that those biocides are not detected by the ALVIM probes.

### 2.2) Detection and removal of biofilm by injection of a biocide

The following experiment has been performed on two parallel channels of the tank. Each one is equipped with an ALVIM probe. Two biocides were tested: sodium hypochlorite (ClO⁻Na⁺) and rocideDB (DBNPA). Batch injections of biocides have been carried out at different development phases of the biofilm (900mV, 1100mV and 1200mV) in order to evaluate the resistance of the biofilm as a function of its maturation time.

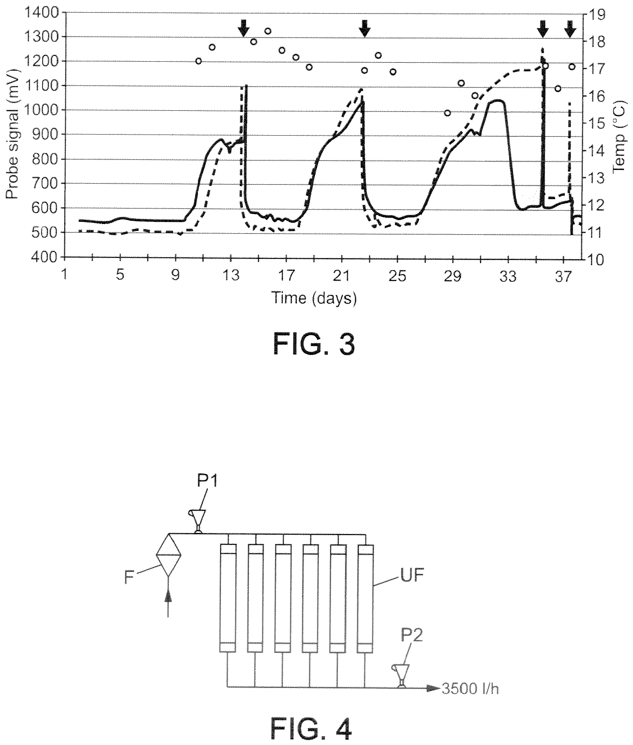

Figure 3 shows the evolution of the probe signal as a function of time. Continuous line represents sodium hypochlorite injection. Dotted line represents DBNPA injection. Dots represent the temperature of the water. Arrows show the time at which the biocide is injected. The probes detect a biofilm growth from the 8^{th} day. The biocide effect of both chemicals is clearly demonstrated at the tested dose. Each injection of biocide brings the signal back to the baseline which corresponds to a clean surface of the probe or at least with a biofilm surface lower than the detection level. Other tests demonstrate that with a continuous injection of 1 mg/L sodium hypochlorite no biofilm growth was detected.

From these results, it can be concluded that:
- the detection of biofilm is repeatable (kinetics do not depend on the channel);
- the monitoring and optimization of a continuous biocide treatment is possible with this equipment;
- the monitoring and optimization of biocide shock treatment is possible with this equipment.

Moreover, regarding the last injection (day 29 - day 37) with a 8-days-old biofilm (dotted curve) it is worth noting that the potential did not reach the baseline (550mV) even one day later. A second injection on day 37 was necessary to get back to the baseline. It shows that the biofilm was not totally removed after the first injection and that part of the biofilm was remaining on the surface of the probe. It is assumed that the biofilm becomes more resistant as the layers accumulate over time. In such conditions, the microorganisms form thicker layers with extracellular material and exopolymers.

Those results highlight even more the importance of a continuous monitoring of biofilm growth in piping and upstream nanofiltration membranes in order to minimize the quantity of chemical cleaning to be applied on the membranes over time. They also show that the probes can alert that a biocide shock treatment was not fully efficient.

### 2.3) Monitoring of the biofilm growth upstream and downstream a membrane fine filtration unit

A second series of tests has been carried out to study the efficiency of inline monitoring upstream and downstream an ultrafiltration unit (pore size 10-50 nm). This type of porous membranes is considered as being able to retain all microorganisms present in natural water.

Ultrafiltration modules are used according to the set up represented at Figure 4. The UF modules are continuously fed with pre-filtered Mediterranean seawater (40µ m multimedia filter F, 2m deep, 50 m from the coast, laminar flowing, Silt Density Index SDI₅ₘᵢₙ = 10-18). The flow rates of the UF modules are: 3000-4000 L/h and the recovery is around 80%.

Two probes are positioned at the following locations of the pilot:
- Probe 1: at the feedwater point of the ultrafiltration module (0.4-0.6 m/s);
- Probe 2: downstream the ultrafiltration module (flowing velocity = 0.3-0.5 m/s).

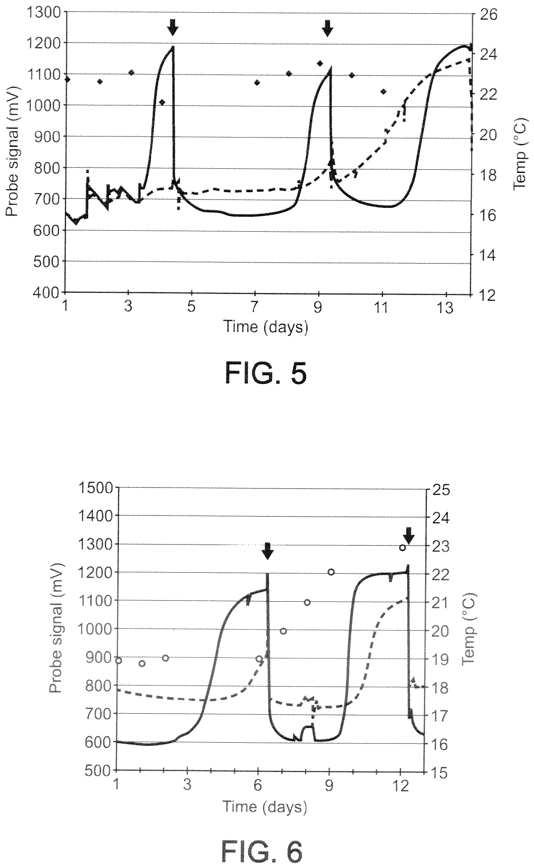

A dose of sodium hypochlorite is injected at various times during the experiment at the inlet of the pilot (upstream probe 1) as shown on Figure 5.

Figure 5 represents the kinetics of biofilm growth on the surface of probe 1 (continuous line) and probe 2 (dotted line). Dots represent the temperature of the water. Arrows show the time at which the dose of sodium hypochlorite is injected (2 ppm). In feedwater the biofilm growth is observed after 4 days approximately whereas in the same time no biofilm growth was observed downstream the ultrafiltration membrane. This can be explained by the fact that microorganisms are totally stopped by the membrane.

When sodium hypochlorite is injected, the signal of the probe 1 goes down rapidly to the baseline twice. During the two sodium hypochlorite injections, the probe 2 (UF permeate) is momentarily removed from the water flow and is consequently not exposed to the biocide. A biofilm growth is observed downstream the membrane by the probe 2 after a total of 10 days. Even if ultrafiltration is able to stop the microorganisms, the permeate is not sterile and a biofilm can still develop even if it takes more than twice longer than with the feed water. This result highlights the importance of biocide strategies to control biofouling of reverse osmosis or nanofiltration when filtering seawater and also freshwater.

Biocide strategies are crucial because reverse osmosis or nanofiltration membranes have a spiral wound configuration and backwash is not possible on such membranes. Once the biofilm is developed, there is no mechanical way to remove the biofouling but only a chemical treatment called cleaning in place.

Figure 6 represents the kinetics of biofilm growth on the surface of probe 2 (dotted line) and probe 1 (continuous line) in steady-state operation. Dots represent the temperature of the water. Arrows show the time at which the dose of sodium hypochlorite is injected (first injection: 50 mg/l for 2 hours; second injection: 50 mg/l for 1 hour). It shows that, under certain conditions, the biofilm can grow faster in the permeate (probe 2) than in the feedwater (probe 1) of the UF installation. This can be partially explained by the difference in water velocity at the location of the probe (feed: 0.17 m/s and permeate: 0.12 m/s). The feedwater probe is thus submitted to higher shear forces that can slow down the biofilm growth rate. The flowing velocity thus has a significant impact on the efficiency of the monitoring system.

These results show that fine filtration units, such as ultrafiltration membranes or microfiltration membranes, do not prevent biofilm growth downstream thereof, even they are theoretically able to physically retain the microorganisms present in water. Therefore, there is need to monitor carefully the development of biofilm downstream those fine filtration units in order to prevent the subsequent biofouling of even finer membranes, such as nanofiltration or reverse osmosis membranes, which may be positioned downstream thereof and which are more difficult to clean up.

## Claims

1. A water injection system for enhanced oil recovery, said water injection system comprising in the direction of water flow:
- a water intake (1) for bringing a flow of water taken from the environment into said water injection system;
- a biocide tank (2) for injecting a dose of biocide into said flow of water,
- a fine filtration unit (4) able to retain particles of a size of 0.1 µm or higher,
- a biofilm sensor (5) in continuous contact with the water flow,
- a water injection pipe (8).

2. The water injection system according to claim 1, wherein said water injection system further comprises a desalination unit (6) located downstream the biofilm sensor (5) and upstream the water injection pipe (8).

3. The water injection system according to claim 2, wherein said desalination unit (6) comprises a desulfation membrane

4. The water injection system according to claim 3, wherein said desulfation membrane unit comprises a nanofiltration membrane, a reverse osmosis membrane or a combination thereof.

5. The water injection system according to any one of claims 1 to 4, wherein said fine filtration unit (4) comprises a media filtration unit, a cartridge filtration unit, a microfiltration membrane or an ultrafiltration membrane, or a combination thereof.

6. The water injection system according to any one of claims 1 to 5, wherein said water injection system further comprises a coarse filter (3) located upstream the filtration unit.

7. The water injection system according to any one of claims 1 to 6, wherein said water injection system further comprises a deaeration unit located upstream or downstream the fine filtration unit (4) or upstream or downstream the desalination unit (6) but always upstream the water injection lines (8).

8. The water injection system according to any one of claims 2 to 7, wherein said water injection system further comprises a biofilm sensor (7) located downstream the desalination unit (6) and upstream the water injection lines (8).

9. The water injection system according to any one of claims 1 to 8, wherein said water injection system is connected to an injection wellhead (10) and a biofilm sensor is located at the injection wellhead (10).

10. The water injection system according to any one of claims 1 to 9, wherein said biofilm sensor (5, 7, 9) is an electrochemical probe, such as an electrochemical probe whose operation is based on the cathodic depolarization induced by the biofilm growth or an electrochemical impedance probe, or a probe operating on differential turbidimetry, light scattering, heat transfer, pressure-drop, real-time measurement of metabolic products, image analysis, radiation signals or electric signal or vibration signals.

11. The water injection system according to any one of claims 1 to 10, wherein said water injection system is an entire subsea system or is placed on a floating vessel or a platform or onshore.

12. A method for removing a biofilm or controlling the formation of a biofilm on the surface of the pipes or the filtering elements of a water injection system for enhanced oil recovery that comprises in the direction of water flow:
- a water intake (1) for bringing a flow of water taken from the environment into said water injection system;
- a biocide tank (2) for injecting a dose of biocide into said the flow of water of said water injection system,
- a fine filtration unit (4) able to retain particles of a size of 0.1 µm or higher,
- a water injection line (8),
wherein said method comprises the following steps:
- providing the water injection system with a biofilm sensor (5) in continuous contact with the water flow, said biofilm sensor (5) being located downstream the fine filtration unit (4),
- measuring the biofilm growth response R of the biofilm sensor (5),
- comparing said biofilm growth response R with a reference value Ro,
- if R is higher than Ro, injecting a dose of biocide into the flow of water from the biocide tank (2 or 11) and/or changing the operating conditions of the fine filtration unit (4).

13. The method according to claim 12, wherein the water injection system further comprises a desalination unit (6) located downstream the biofilm sensor (5) and upstream the water injection lines (8).

14. The method according to claim 13, wherein said desalination unit (6) comprises a desulfation membrane.

15. The method according to claim 14, wherein said desulfation membrane comprises a nanofiltration membrane or a reverse osmosis membrane.

16. The method according to any one of claims 12 to 15, wherein said fine filtration unit (4) comprises a media filtration unit, a cartridge filtration unit, a microfiltration membrane or an ultrafiltration membrane, or a combination thereof.

17. The method according to any one of claims 12 to 16, wherein said water injection system further comprises coarse filter(s) (3) located upstream the fine filtration unit (4).

18. The method according to any one of claims 13 to 17, wherein said water injection system further comprises a deaeration unit located downstream the fine filtration unit (4) and upstream the desalination unit (6) or downstream the desalination unit (6) and upstream the water injection lines (8).

19. The method according to any one of claims 13 to 18, further comprising the following steps:
- providing the water injection system with a further biofilm sensor (7) located downstream the desalination unit (6) and upstream the water injection line (8),
- measuring the biofilm growth response Ra of the further biofilm sensor (7),
- comparing said biofilm growth response Ra with a reference value Rao,
- if Ra is higher than Rao, injecting a dose of biocide into the flow of water from the biocide tank (2, 11 or 12) and/or changing the operating conditions of the fine filtration unit (4) and/or the desalination unit (6).

20. The method according to any one of claims 12 to 19, wherein said water injection system is connected to an injection wellhead (10) and said method further comprises:
- providing the water injection system with a further biofilm sensor (9) located at the injection wellhead (10),
- measuring the biofilm growth response Rb of the further biofilm sensor (9),
- comparing said biofilm growth response Rb with a reference value Rbo,
- if Rb is higher than Rbo, injecting a dose of biocide into the flow of water from the biocide tank (2, 11, 12, 13 or 14) and/or changing the operating conditions of the fine filtration unit (4) and/or the desalination unit (6).

21. The method according to any one of claims 12 to 20, wherein said biofilm sensor (5, 7, 9) is an electrochemical probe, such as an electrochemical probe whose operation is based on the cathodic depolarization induced by the biofilm growth or an electrochemical impedance probe, or a probe operating on differential turbidimetry, light scattering, heat transfer, pressure-drop, real-time measurement of metabolic products, image analysis, radiation signals or electric signal or vibration signals.

## Patentansprüche

1. Wassereinspritzsystem zur verbesserten Ölrückgewinnung, wobei das Wassereinspritzsystem in der Richtung des Wasserstroms umfasst:
- einen Wassereinlass (1), zum Einbringen eines Wasserstroms, der aus der Umgebung entnommen wird, in das Wassereinspritzsystem;
- einen Biozidtank (2) zum Einspritzen einer Dosis an Biozid in den Wasserstrom,
- eine Feinfiltrationseinheit (4), die Partikel mit einer Größe von 0,1 µm oder größer zurückhalten kann,
- einen Biofilmsensor (5) in ständigem Kontakt mit dem Wasserstrom,
- ein Wassereinspritzrohr (8).

2. Wassereinspritzsystem nach Anspruch 1, wobei das Wassereinspritzsystem weiterhin eine Entsalzungseinheit (6) umfasst, die stromabwärts des Biofilmsensors (5) und stromaufwärts des Wassereinspritzrohrs (8) lokalisiert ist.

3. Wassereinspritzsystem nach Anspruch 2, wobei die Entsalzungseinheit (6) eine Desulfatierungsmembran umfasst.

4. Wassereinspritzsystem nach Anspruch 3, wobei die Desulfatierungsmembraneinheit eine Nanofiltrationsmembran, eine Umkehrosmosemembran oder eine Kombination davon umfasst.

5. Wassereinspritzsystem nach einem der Ansprüche 1 bis 4, wobei die Feinfiltrationseinheit (4) eine Medienfiltrationseinheit, eine Patronenfiltrationseinheit, eine Mikrofiltrationsmembran oder eine Ultrafiltrationsmembran oder eine Kombination davon umfasst.

6. Wassereinspritzsystem nach einem der Ansprüche 1 bis 5, wobei das Wassereinspritzsystem weiterhin einen Grobfilter (3) umfasst, der stromaufwärts der Filtrationseinheit lokalisiert ist.

7. Wassereinspritzsystem nach einem der Ansprüche 1 bis 6, wobei das Wassereinspritzsystem weiterhin eine Entlüftungseinheit umfasst, die stromaufwärts oder stromabwärts der Feinfiltrationseinheit (4) oder stromaufwärts oder stromabwärts der Entsalzungseinheit (6), jedoch immer stromaufwärts der Wassereinspritzleitungen (8) lokalisiert ist.

8. Wassereinspritzsystem nach einem der Ansprüche 2 bis 7, wobei das Wassereinspritzsystem weiterhin einen Biofilmsensor (7) umfasst, der stromabwärts der Entsalzungseinheit (6) und stromaufwärts der Wassereinspritzleitungen (8) lokalisiert ist.

9. Wassereinspritzsystem nach einem der Ansprüche 1 bis 8, wobei das Wassereinspritzsystem mit einem Einspritzbohrkopf (10) verbunden ist und ein Biofilmsensor am Einspritzbohrkopf (10) lokalisiert ist.

10. Wassereinspritzsystem nach einem der Ansprüche 1 bis 9, wobei der Biofilmsensor (5, 7, 9) eine elektrochemische Sonde ist, wie zum Beispiel eine elektrochemische Sonde, deren Betrieb auf der kathodischen Depolarisation beruht, die durch das Biofilmwachstum induziert wird, oder eine elektrochemische Impedanzsonde, oder eine Sonde, die mit Differentialturbidimetrie, Lichtstreuung, Wärmeübertragung, Druckabfall, Echtzeitmessung von Stoffwechselprodukten, Bildanalyse, Strahlungssignalen oder elektrischem Signal oder Vibrationssignalen arbeitet.

11. Wassereinspritzsystem nach einem der Ansprüche 1 bis 10, wobei das Wassereinspritzsystem ein gesamtes Unterwassersystem ist oder auf einem schwimmenden Schiff oder einer Plattform oder an Land platziert ist.

12. Verfahren zur Entfernung eines Biofilms oder zur Steuerung der Bildung eines Biofilms auf der Oberfläche der Rohre oder der Filterelemente eines Wassereinspritzsystems zur verbesserten Ölrückgewinnung, das in der Richtung des Wasserstroms umfasst:
- einen Wassereinlass (1) zum Einbringen eines Wasserstroms, der aus der Umgebung entnommen wird, in das Wassereinspritzsystem;
- einen Biozidtank (2) zum Einspritzen einer Dosis an Biozid in den Wasserstrom des Wassereinspritzsystems;
- eine Feinfiltrationseinheit (4), die Partikel mit einer Größe von 0,1 µm oder größer zurückhalten kann;
- eine Wassereinspritzleitung (8),
wobei das Verfahren die folgenden Schritte umfasst:
- Bereitstellen des Wassereinspritzsystems mit einem Biofilmsensor (5) in ständigem Kontakt mit dem Wasserstrom, wobei der Biofilmsensor (5) stromabwärts der Feinfiltrationseinheit (4) lokalisiert ist,
- Messen der Biofilmwachstumsantwort R des Biofilmsensors (5),
- Vergleichen der Biofilmwachstumsantwort R mit einem Referenzwert Ro,
- wenn R höher als Ro ist, Einspritzen einer Dosis an Biozid in den
Wasserstrom aus dem Biozidtank (2 oder 11), und/oder Ändern der Betriebsbedingungen der Feinfiltrationseinheit (4).

13. Verfahren nach Anspruch 12, wobei das Wassereinspritzsystem weiterhin eine Entsalzungseinheit (6) umfasst, die stromabwärts des Biofilmsensors (5) und stromaufwärts der Wassereinspritzleitungen (8) lokalisiert ist.

14. Verfahren nach Anspruch 13, wobei die Entsalzungseinheit (6) eine Desulfatierungsmembran umfasst.

15. Verfahren nach Anspruch 14, wobei die Desulfatierungsmembran eine Nanofiltrationsmembran oder eine Umkehrosmosemembran umfasst.

16. Verfahren nach einem der Ansprüche 12 bis 15, wobei die Feinfiltrationseinheit (4) eine Medienfiltrationseinheit, eine Patronenfiltrationseinheit, eine Mikrofiltrationsmembran oder eine Ultrafiltrationsmembran oder eine Kombination davon umfasst.

17. Verfahren nach einem der Ansprüche 12 bis 16, wobei das Wassereinspritzsystem weiterhin (einen) Grobfilter (3) umfasst, der/die stromaufwärts der Feinfiltrationseinheit (4) lokalisiert ist/sind.

18. Verfahren nach einem der Ansprüche 13 bis 17, wobei das Wassereinspritzsystem weiterhin eine Entlüftungseinheit umfasst, die stromabwärts der Feinfiltrationseinheit (4) und stromaufwärts der Entsalzungseinheit (6) oder stromabwärts der Entsalzungseinheit (6) und stromaufwärts der Wassereinspritzleitungen (8) lokalisiert ist.

19. Verfahren nach einem der Ansprüche 13 bis 18, weiterhin umfassend die folgenden Schritte:
- Bereitstellen des Wassereinspritzsystems mit einem weiteren Biofilmsensor (7), der stromabwärts der Entsalzungseinheit (6) und stromaufwärts der Wassereinspritzleitung (8) lokalisiert ist;
- Messen der Biofilmwachstumsantwort Ra des weiteren Biofilmsensors (7),
- Vergleichen der Biofilmwachstumsantwort Ra mit einem Referenzwert Rao,
- wenn Ra höher als Rao ist, Einspritzen einer Dosis an Biozid in den Wasserstrom aus dem Biozidtank (2, 11 oder 12) und/oder Ändern der Betriebsbedingungen der Feinfiltrationseinheit (4) und/oder der Entsalzungseinheit (6).

20. Verfahren nach einem der Ansprüche 12 bis 19, wobei das Wassereinspritzsystem mit einem Einspritzbohrkopf (10) verbunden ist und wobei das Verfahren weiterhin umfasst:
- Bereitstellen des Wassereinspritzsystems mit einem weiteren Biofilmsensor (9), der an dem Einspritzbohrkopf (10) lokalisiert ist,
- Messen der Biofilmwachstumsantwort Rb des weiteren Biofilmsensors (9),
- Vergleichen der Biofilmwachstumsantwort Rb mit einem Referenzwert Rbo,
- wenn Rb höher als Rbo ist, Einspritzen einer Dosis an Biozid in den Wasserstrom aus dem Biozidtank (2, 11, 12, 13 oder 14) und/oder Ändern der Betriebsbedingungen der Feinfiltrationseinheit (4) und/oder der Entsalzungseinheit (6).

21. Verfahren nach einem der Ansprüche 12 bis 20, wobei der Biofilmsensor (5, 7, 9) eine elektrochemische Sonde ist, wie zum Beispiel eine elektrochemische Sonde, deren Betrieb auf der kathodischen Depolarisation beruht, die durch das Biofilmwachstum induziert wird, oder eine elektrochemische Impedanzsonde, oder eine Sonde, die mit Differentialturbidimetrie, Lichtstreuung, Wärmeübertragung, Druckabfall, Echtzeitmessung von Stoffwechselprodukten, Bildanalyse, Strahlungssignalen oder elektrischem Signal oder Vibrationssignalen arbeitet.

## Revendications

1. Système d'injection d'eau pour une récupération assistée du pétrole, ledit système d'injection d'eau comprenant dans la direction d'écoulement d'eau :
- une prise d'eau (1) pour amener un écoulement d'eau pris de l'environnement dans ledit système d'injection d'eau ;
- un réservoir de biocide (2) pour injecter une dose de biocide dans ledit écoulement d'eau,
- une unité de filtration fine (4) apte à retenir des particules d'une taille supérieure ou égale à 0,1 µm,
- un capteur de biofilm (5) en contact continu avec l'écoulement d'eau,
- un tuyau d'injection d'eau (8).

2. Système d'injection d'eau selon la revendication 1, dans lequel ledit système d'injection d'eau comprend en outre une unité de dessalement (6) située en aval du capteur de biofilm (5) et en amont du tuyau d'injection d'eau (8).

3. Système d'injection d'eau selon la revendication 2, dans lequel ladite unité de dessalement (6) comprend une membrane de désulfatation.

4. Système d'injection d'eau selon la revendication 3, dans lequel ladite unité de membrane de désulfatation comprend une membrane de nanofiltration, une membrane d'osmose inversée ou une combinaison de celles-ci.

5. Système d'injection d'eau selon l'une quelconque des revendications 1 à 4, dans lequel ladite unité de filtration fine (4) comprend une unité de filtration à support, une unité de filtration à cartouche, une membrane de microfiltration ou une membrane d'ultrafiltration, ou une combinaison de celles-ci.

6. Système d'injection d'eau selon l'une quelconque des revendications 1 à 5, dans lequel ledit système d'injection d'eau comprend en outre un filtre grossier (3) situé en amont de l'unité de filtration.

7. Système d'injection d'eau selon l'une quelconque des revendications 1 à 6, dans lequel ledit système d'injection d'eau comprend en outre une unité de désaération située en amont ou en aval de l'unité de filtration fine (4) ou en amont ou en aval de l'unité de dessalement (6) mais toujours en amont des conduites d'injection d'eau (8).

8. Système d'injection d'eau selon l'une quelconque des revendications 2 à 7, dans lequel ledit système d'injection d'eau comprend en outre un capteur de biofilm (7) situé en aval de l'unité de dessalement (6) et en amont des conduites d'injection d'eau (8).

9. Système d'injection d'eau selon l'une quelconque des revendications 1 à 8, dans lequel ledit système d'injection d'eau est raccordé à une tête de puits d'injection (10) et un capteur de biofilm est situé à la tête de puits d'injection (10).

10. Système d'injection d'eau selon l'une quelconque des revendications 1 à 9, dans lequel ledit capteur de biofilm (5, 7, 9) est une sonde électrochimique, comme une sonde électrochimique dont le fonctionnement est basé sur la dépolarisation cathodique induite par la croissance de biofilm ou une sonde d'impédance électrochimique, ou une sonde fonctionnant sur une turbidimétrie différentielle, une diffusion de la lumière, un transfert de chaleur, une baisse de pression, une mesure en temps réel de produits métaboliques, une analyse d'image, des signaux de rayonnement ou un signal électrique ou des signaux de vibrations.

11. Système d'injection d'eau selon l'une quelconque des revendications 1 à 10, dans lequel ledit système d'injection d'eau est un système entièrement sous-marin ou est placé sur un navire flottant ou sur une plate-forme ou à terre.

12. Procédé d'enlèvement d'un biofilm ou de commande de la formation d'un biofilm sur la surface des tuyaux ou des éléments de filtration d'un système d'injection d'eau pour une récupération assistée du pétrole comprenant dans la direction d'écoulement d'eau :
- une prise d'eau (1) pour amener un écoulement d'eau pris de l'environnement dans ledit système d'injection d'eau ;
- un réservoir de biocide (2) pour injecter une dose de biocide dans ledit écoulement d'eau dudit système d'injection d'eau,
- une unité de filtration fine (4) apte à retenir des particules d'une taille supérieure ou égale à 0,1 µm,
- une conduite d'injection d'eau (8),
dans lequel ledit procédé comprend les étapes suivantes :
- la fourniture, au système d'injection d'eau, d'un capteur de biofilm (5) en contact continu avec l'écoulement d'eau, ledit capteur de biofilm (5) étant situé en aval de l'unité de filtration fine (4),
- la mesure de la réponse de croissance de biofilm R du capteur de biofilm (5),
- la comparaison de ladite réponse de croissance de biofilm R à une valeur de référence Ro,
- si R est supérieur à Ro, l'injection d'une dose de biocide dans l'écoulement d'eau depuis le réservoir de biocide (2 ou 11) et/ou le changement des conditions de fonctionnement de l'unité de filtration fine (4).

13. Procédé selon la revendication 12, dans lequel le système d'injection d'eau comprend en outre une unité de dessalement (6) située en aval du capteur de biofilm (5) et en amont des conduites d'injection d'eau (8).

14. Procédé selon la revendication 13, dans lequel ladite unité de dessalement (6) comprend une membrane de désulfatation.

15. Procédé selon la revendication 14, dans lequel ladite membrane de désulfatation comprend une membrane de nanofiltration ou une membrane d'osmose inversée.

16. Procédé selon l'une quelconque des revendications 12 à 15, dans lequel ladite unité de filtration fine (4) comprend une unité de filtration à support, une unité de filtration à cartouche, une membrane de microfiltration ou une membrane d'ultrafiltration, ou une combinaison de celles-ci.

17. Procédé selon l'une quelconque des revendications 12 à 16, dans lequel ledit système d'injection d'eau comprend en outre un ou plusieurs filtres grossiers (3) situés en amont de l'unité de filtration fine (4).

18. Procédé selon l'une quelconque des revendications 13 à 17, dans lequel ledit système d'injection d'eau comprend en outre une unité de désaération située en aval de l'unité de filtration fine (4) et en amont de l'unité de dessalement (6) ou en aval de l'unité de dessalement (6) et en amont des conduites d'injection d'eau (8).

19. Procédé selon l'une quelconque des revendications 13 à 18, comprenant en outre les étapes suivantes :
- la fourniture, au système d'injection d'eau, d'un autre capteur de biofilm (7) situé en aval de l'unité de dessalement (6) et en amont de la conduite d'injection d'eau (8),
- la mesure de la réponse de croissance de biofilm Ra de l'autre capteur de biofilm (7),
- la comparaison de ladite réponse de croissance de biofilm Ra à une valeur de référence Rao,
- si Ra est supérieur à Rao, l'injection d'une dose de biocide dans l'écoulement d'eau depuis le réservoir de biocide (2, 11 ou 12) et/ou le changement des conditions de fonctionnement de l'unité de filtration fine (4) et/ou de l'unité de dessalement (6).

20. Procédé selon l'une quelconque des revendications 12 à 19, dans lequel ledit système d'injection d'eau est raccordé à une tête de puits d'injection (10) et ledit procédé comprend en outre :
- la fourniture, au système d'injection d'eau, d'un autre capteur de biofilm (9) situé à la tête de puits d'injection (10),
- la mesure de la réponse de croissance de biofilm Rb de l'autre capteur de biofilm (9),
- la comparaison de ladite réponse de croissance de biofilm Rb à une valeur de référence Rbo,
- si Rb est supérieur à Rbo, l'injection d'une dose de biocide dans l'écoulement d'eau depuis le réservoir de biocide (2, 11, 12, 13 ou 14) et/ou le changement des conditions de fonctionnement de l'unité de filtration fine (4) et/ou de l'unité de dessalement (6).

21. Procédé selon l'une quelconque des revendications 12 à 20, dans lequel ledit capteur de biofilm (5, 7, 9) est une sonde électrochimique, comme une sonde électrochimique dont le fonctionnement est basé sur la dépolarisation cathodique induite par la croissance de biofilm ou une sonde d'impédance électrochimique, ou une sonde fonctionnant sur une turbidimétrie différentielle, une diffusion de la lumière, un transfert de chaleur, une baisse de pression, une mesure en temps réel de produits métaboliques, une analyse d'image, des signaux de rayonnement ou un signal électrique ou des signaux de vibrations.
